(19) Europäisches Patentamt
European Patent Office
Office européen des brevets

(11) Publication number: **0 126 866**
**A1**

# (12) EUROPEAN PATENT APPLICATION

(21) Application number: 84102448.2

(22) Date of filing: 07.03.84

(51) Int. Cl.³: C 08 F 20/36
C 08 F 299/04, C 07 C 93/193
A 61 K 6/08

(30) Priority: 26.04.83 US 486688

(43) Date of publication of application: 05.12.84 Bulletin 84/49

(84) Designated Contracting States: AT BE CH DE FR GB IT LI LU NL SE

(71) Applicant: DENTSPLY INTERNATIONAL, INC.
570 West College Avenue
York Pennsylvania 17405(US)

(72) Inventor: Tateosian, Louis Hagop
209 Reynolds Mill Road
York Pennsylvania 17403(US)

(72) Inventor: Horne, Shek Chee
P.O. Box 523
Somers, Conn. 06071(US)

(74) Representative: Wächtershäuser, Günter, Dr.
Tal 29
D-8000 München 2(DE)

## (54) Organic amine salt of an acid, manufacture and use as accelerator.

(57) The present application pertains to a new organic amine salt of an acid. The use of organic amine salts of acids as photoaccelerators is especially useful in visible light photopolymerization when speed and depth of cure are needed. The preferred organic amine salt is of an organic acid with both the salt and amine moieties being unsaturated, the acid being methacrylic acid and the amine NN(dimethyl)-3-neopentyl acrylate. A new method for preparing the organic amine salt is also shown involving cooling both the organic amine and the acid before they are brought together in a non-aqueous reaction.

EP 0 126 866 A1

# ORGANIC AMINE SALT OF AN ACID, MANUFACTURE AND USE AS ACCELERATOR

## BACKGROUND OF THE INVENTION

The present invention relates to a new organic amine salt of an acid, the manufacture of such a salt and broadly the use of organic amine salts as polymerization accelerators particularly in visible light curing systems.

It is known, see 4,071,424, that amines function as photoaccelerators in light curing compositions employing diketone photoinitiators to polymerize ethylenically unsaturated organic compounds. It is also known to use betaine salts which are also based on organic amines as photopolymerizable monomer, see 4,297,185. Betaine should be contrasted with the organic amine salt of the present invention in which the acid is displaceable by a strong base to regenerate amine. In betaine which is the addition product of an amine to an $\alpha, \beta$ - unsaturated carboxylic acid, the betaine amine is not displaceable by a strong base according to current understanding.

An object of the present invention is to provide a polymerizable composition that has a rapid rate of cure and a substantial depth of cure capability even with highly opaque compositions.

Another object of the present invention is to provide a photoaccelerator having low volatility and toxicity to enhance health advantages.

Yet another object of the invention is to provide a polymerizable composition having good color stability on aging and low odor.

Another object of the invention is to provide a superior degree of polymerization.

Another object of the invention is to provide an accelerator that is copolymerizable and highly resistant to extraction.

A further object of the invention is the provision of a new improved organic amine salt compound.

Another object of the invention is to provide a method of producing the new improved organic amine salt compound of the present invention.

SUMMARY OF THE INVENTION

The present invention provides a new method of modifying polymerizable compositions by introducing a new polymerization modifying system that is a neopentyl acrylate amine moiety and an acid moiety. The particularly preferred modifying system is one in which the recited amine moiety and acid moiety are associated in a salt and preferably the acid moiety is a methacrylic acid.

The invention also provides a new superior polymerizable composition that contains in addition to the initiator an organic amine salt compound. The organic amine salt acts as an accelerator and preferably has unsaturation in both its amine and acid moiety. Most preferably the polymerizable composition is one that is curabl by visible light radiation and is a dental composition suitable for use in the oral environment.

By another aspect, the invention provides a new composition of matter that is a salt having the formula

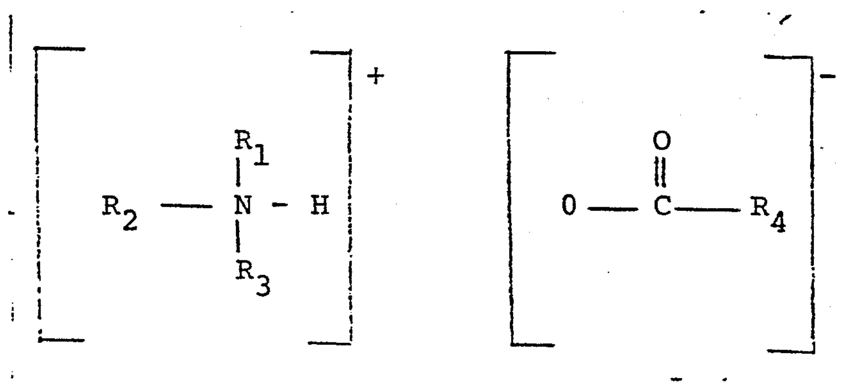

wherein $R_1$, $R_2$ and $R_3$ are each either an organic radical or H and at least one of $R_1$, $R_2$ and $R_3$ contains the group

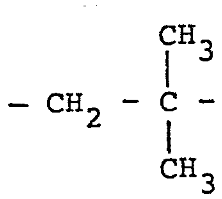

and $R_4$ is an organic radical or H.

By yet another feature of the invention, a new method is provided for producing organic amine salts of acids which involves cooling at least the organic amine or acid feed stock to below 0°C before they are brought together for reaction. Preferably both feed stocks are cooled to below 0°C and preferably the acid is frozen solid.

DESCRIPTION OF THE PREFERRED EMBODIMENT

The present invention in its preferred embodiment is a photoaccelerated polymerizable composition employing a new organic amine salt compound with a conventional polymerization initiator compound. Broadly, it has been determined that the salts of the organic amines are superior photoaccelerators to the organic amines themselves at least in a diverse variety of situations and that in the other situations the salts of the organic amines operate to modify the polymerization of diverse polymerizable compositions in ways differing from the organic amines themselves. As used in this patent application, the term salt of an organic amine does not include the betaineswhich are contrasted as set forth in the BACKGROUND OF THE INVENTION section of this application.

The organic amines that are applicable to the present invention are all inclusive of the alkyl and substituted alkyl amines, but those preferred are represented by the formula:

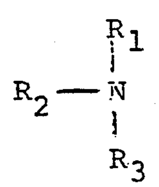

wherein $R_1$, $R_2$ and $R_3$ are each either an organic radical or H (hydrogen atom). R with a subscript represents a radical. N represents a nitrogen atom.

The more preferred amines are the tertiary amines. The amines that are most preferred are those with a neopentyl acrylate amine moiety and especially amines having the neopentyl acrylate moiety that are tertiary amines and most preferable those that are represented by the formula:

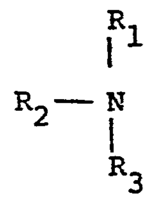

wherein $R_1$ and $R_2$ are alkyl or substituted alkyl radicals having from 1-4 C (carbon atoms) and $R_3$ is a neopentyl acrylate radical having a total of 8 to 10 C. More preferably $R_1$ and $R_2$ are methyl and $R_3$ is a neopently acrylate radical having 8 C.

A neopently acrylate radical having 8 C is represented by the formula:

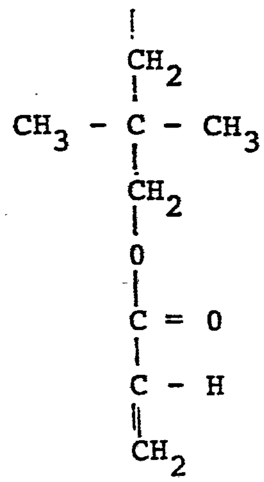

By a neopently acrylate radical having 8 to 10 C it is meant not only the acrylate (as depicted in the formula above), but also the methacrylate or ethacrylate. Other neopentyl radicals that may be preferred in some special situations might be saturated, as well as unsaturated. 0 represents an oxygen atom.

The acids that are applicable to the present invention are inclusive of all compounds having an acid moiety but those preferred are the organic acids.

The more preferred classes of acids exclude the strong organic acid, such as the oxalic, picric, sulfonilic, triflu-romethane sulfonic, and perfluorobutyric acids.

The most preferred organic acids are those having mono acid functionality that are represented by the formula:

$$H-O-\overset{\overset{\large O}{\|}}{C}-R_4$$

wherein $R_4$ is an organic radical or H, more preferably $R_4$ is unsaturated and preferably contains a vinyl group and has from 3 to 6 C, $R_4$ is more preferably

$$-\overset{\overset{\large CH_3}{|}}{C}=CH_2$$

When $R_4$ is given as vinyl having from 3 to 6 C it is meant that the acid may be not only acrylic or propenoic acid but also methacrylic acid, ethacrylic acid and propylacrylic acid.

As already mentioned in the preferred form of the present invention, the organic amine moiety and the acid moiety are first associated in a salt. The organic amine salt compound is combined with a polymerization initiator compound a polymerizable composition. The preferred salts are represented by the formula:

$$\left[R_2-\overset{\overset{\large R_1}{\cdot}}{\underset{\underset{\large R_3}{\cdot}}{N}}-H\right]^{+}\left[O-\overset{\overset{\large O}{\cdot}}{C}-R_4\right]^{-}$$

wherein $R_1$, $R_2$, $R_3$ and $R_4$ have the meanings previously given for them. It will be understood that the preferred salts giving superior efficacy are those previously identified by their amine and acid constituancy as being more and most preferred in the order previously indicated.

The organic amine salt compounds preferably are unsaturated via $R_1$, $R_2$ or $R_3$ and $R_4$, thus providing the capability of themselves forming a polymer. More preferably, the polymerizable composition contains at least one polymerizable organic compound other than the organic amine salt compound. Thus, in its preferred embodiment and its greatest presently contemplated

utility, the preferred organic amine salt is included in a composition with at least one polymerizable organic compound other than the organic amine salt. The organic amine salt can be chosen from the group consisting of polymerizable and unpolymerizable organic amine salts and mixtures thereof.

The polymerizable organic compound excluding the amine salt compound is inclusive of all organic compounds and combinations of compounds that are polymerizable but those preferred are the ethylenically unsaturated compounds, which should be free-radical polymerizable. A suitable compound is a monomer containing ethylenic unsaturation in a terminal group. For example, the ethylenically unsaturated material may be one or more monomers selected from vinyl monomers, allyl monomers and vinylidene monomers. Suitable vinyl monomers which may be polymerized include, for example, vinyl esters, aromatic vinyl compounds and vinyl nitriles. Vinyl esters suitable for use in the method of our invention include, for example, vinyl acetate.

Preferably, the predominant polymerizable composition is an acrylate polymerized predominantly through addition polymerization, more preferable one chosen from the group consisting of mono and multifunctional acrylates. By acrylate as used in this phraseology, it is meant to include the acrylates and methacrylates. Especially preferred compounds are the multifunctional acrylates such as 1, 6-hexandiol dimethacrylate and the urethane dimethacrylate oligomers.

By at least one polymerizable organic compound, it will be understood in this application to mean one or more such compounds.

The preferred polymerization initiator compound is a radiation activatable compound. In some instances, a heat

activatable initiator compound may also be included. More preferably, the radiation activatable compound is an actinic light activatable compound. Such compounds are well-known, see, for example, U.S. Patent No. 4,245,031, the contents of which are incorporated herein by reference.

Most preferably, the initiator is a visible light activatable compound making the polymerizable composition curable with visible light. The preferred class of initiators is the well-known diketones and the most preferred is camphoroquinone. Visible light activatable photoinitiators are discussed in U.S. Patent No. 4,110,184, the contents of which are incorporated herein by reference.

The organic amine salt compound, the radiation activatable compound and the at least one polymerizable organic compound (one or more polymerizable organic compounds in addition to the organic amine salt compound or compounds) constitute the polymerizable system. The organic amine salt compound is preferably present in the polymerizable system in an amount of 0.05 to 99 percent by weight of the polymerizable system constituents, more preferably in an amount of 0.1 to 10 percent and most preferably in an amount of 0.3 to 5 percent. The radiation activatable compound is preferably present in the polymerizable system in an amount of 0.001 to 10 percent by weight of the polymerizable system constituents, more preferably 0.01 to 5 percent and most preferably 0.1 to 0.5 percent. The at least one polymerizable organic compound is preferably present in the polymerizable system in an amount of 1.0 to 99.9 percent by weight of the polymerizable system constituents, more preferably 85 to 99.9 percent and most preferably 94.5 to 99.6 percent.

In addition to the primary polymerization active constitutients of the composition discussed above, large quantities of other ingredients may be present, for example, the silaneous fillers and other fillers by way of example apatite, soda glass, quartz, silica gel, borosilicate glass, alumina, metal fibres and particulate polymer. These may be in the form of spheres, platelets, fibres, whiskers or they may be irregularly shaped.

Many other constitutients may also be present, such as stabilizing agents, plasticizers, inert diluents, solvents, pigments, opaquing agents, etc. Such materials are discussed in the previously referenced two patents. Such teachings are well-known and their repetition is therefore unnecessary. In some embodiments, substantial filler may be present and it is preferred to have a filler content of 0 to 70 percent of the polymerizable composition on a volume basis based on total volume of the polymerizable composition, and in many filled compositions where the present invention has special significance in enabling adequate cure, 35 to 70 percent and even 45 to 70 percent.

An important feature of the present invention is the provision of a system in its preferred forms that will cure in response to visible light quickly to a high degree of cure and to substantial depths. This highly chemically reactive photoresponsive material is storage stable at 20°C for at least one week under light-free conditions in its preferred forms, and can achieve storage stability of more than a year.

The invention provides by the recited means a method of modifying the polymerization of polymerizable compositions. These means having been recited in detail in the discussion of the compositions will not be repeated here as the method of the means because this is readily apparent from the discussion already

THE METHOD OF FORMING THE ORGANIC AMINE SALT

While the organic amine salt of an acid may be formed by other means, the preferred method and the method of the present invention that will produce an amine salt of an acid is given below. By an organic amine salt as used herein, it is meant as previously stated, the salt as described earlier in this application and as contrasted with betaines in the BACKGROUND OF THE INVENTION section of this application.

First, separate feed stocks of an organic amine and an acid are formed. These feed stocks are then brought together under conditions such that one of the combined feed stocks is below 0°C at the time of combining. Preferably both the organic amine feed stock and the acid feed stock are cooled to at least 0°C before the feed stocks are combined. More preferably, the acid is an organic acid and the cooling is to a degree that solidifies the organic acid and the organic acid and organic amine are brought together in reaction with the organic acid initially in a solid state and the reaction is exothermic. The preferred organic amines and the preferred organic acid reactants are those previously described in this application with the preferred and previously determined efficacies of this invention being those previously indicated. Preferably the organic amine salt is formed under nonaqueous conditions.

As disclosed in Example 2, small quantities of the organic amine salt can be prepared conveniently without cooling. This is believed to be the situation because the very small quantity does not heat excessively during the exotherm of the reaction.

The invention is further illustrated by the following examples:

EXAMPLE 1

An organic amine salt of an acid was formed by preparing two feed stocks as follows:

92.8 g glacial methacrylic acid (MAA) (99.3% purity, with 250 ppm methyl ether hydroquinone [MEHQ]) was charged to an amber glass bottle. 0.28 g buylated hydroxytoluene (BHT) was added to the MAA and shaken until dissolved into a uniform solution. The MAA solution was placed into a freezer set at -12°C for about one hour. The solution became a frozen solid.

At substantially the same time, about 200 g of 3-dimethylaminoneopentyl acrylate (DMANPA) also known as 3-(N,N-dimethylamino)-2,2-dimethylpropyl acrylate (99% purity with 50 ppm MEHQ) was charged to an amber glass bottle and placed in the freezer set at -12°C for about one hour. The DMANPA did not freeze and remained liquid.

Both bottles were removed from the freezer and 185 g DMANPA was poured onto the frozen MAA solid. The combined MAA and DMANPA were then shaken vigorously on a mechanical shaker. The exotherm of the salt reaction melted the frozen MAA and the amine salt formed as a liquid. Shaking was continued for 10 minutes to ensure complete reaction.

A slight stochiometric excess of MAA was used for odor modification in Example 1 to compensate for the impurities inherently present in the starting materials. The theoretical molar equivalent for DMANPA-MAA amine salt is 185/86 (DMANPA/MAA). Experience indicates that a DMANPA/MAA ratio lower than 185/95 tends to slow down the photoaccelerators efficiency.

EXAMPLE 2

Another 1:1 mole ratio acid amine salt reaction product was formed by reacting 8.6 g of MAA and 15.7 g of dimethylaminoethyl methacrylate (DMAEMA) by combining these small quantities at room temperature in a 2 oz amber glass container at room temperature and shaking them on a mechanical shaker for about one half hour and then letting stand for about one hour before using. It is believed that the cooling is not necessary because of the small quantities involved. The resulting amine salt DMAEMA-MAA was a clear, slightly viscous liquid.

To determine the nature of the compound formed, infrared spectra were run on glacial methacrylic acid (MAA), dimethylaminoethyl methacrylate (DMAEMA) and the above reaction product (DMAEMA-MAA). The spectra confirm that the DMAEMA-MAA salt is a new compound and not just a simple physical mixture of MAA and DMAEMA.

The differences of the spectra are very obvious and can be explained as follows:

I. Disappearance of Free -COOH Group

The DMAEMA-MAA salt has an entirely new form in the 2500-3300 $cm^{-1}$ region. The broad and strong -COOH band (due to -OH vibration) of MAA in this region changed after the reaction. Noticeable also is the disappearance of the strong -OH vibration at 2650 $cm^{-1}$, 2750 $cm^{-1}$, and 1750 $cm^{-1}$.

In addition, the C-O vibrations in MAA (at 1715 $cm^{-1}$) and DMAEMA (1740 $cm^{-1}$) have been shifted to 1725 $cm^{-1}$ in the new salt.

II. Formation of $-COO^{-}$ $>NH^{+}$ Salt

The new bands formed in DMAEMA-MAA spectra in the 2400-2600 $cm^{-1}$ region and the 1850-2050 $cm^{-1}$ region are indications of the new $-COO^{-}$ $>NH^{+}$ salt formation.

The new band in the 1550-1620 $cm^{-1}$ region and the higher intensity band at 1410 $cm^{-1}$ also indicated the formation of $-COO^{-}$ salt, or $-CH_2-N^{+}<$ compounds.

Additionally, the missing of 1115 $cm^{-1}$ band (due to C-N stretching) in DMAEMA after the reaction indicated the nature of the C-N bond has been changed.

In conclusion, the I.R. spectra evidenced the following believed reaction between DMAEMA and MAA:

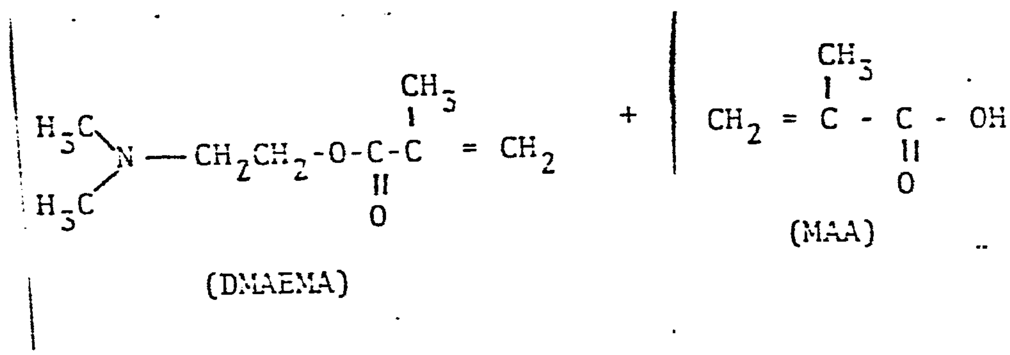

(DMAEMA) (MAA)

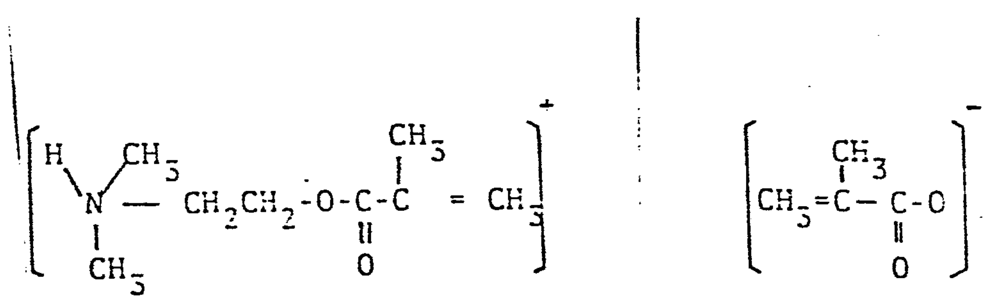

(DMAEMA-MAA salt)

EXAMPLE 3

Another experiment was carried out to demonstrate the nature of the reaction product of the DMAEMA-MAA reaction prepared according to Example 2. The volatility of the DMAEMA, MAA and the reaction product was measured by placing 5 grams of each of the three in 10 ml bottles and storing them open for six days at room temperature. The following weight changes were observed:

| | | |
|---|---|---|
| pure Methacrylic acid | - | weight loss 5.0% |
| pure DMAEMA | - | weight loss 4.6% |
| DMAEMA-MAA salt | - | weight gain 2.0% |

Both MAA and DMAEMA showed vapor loss during open storage. Surprisingly, the amine-acid salt (DMAEMA-MAA) showed no vapor loss during open storage. Instead, it gained some weight. The lower vapor pressure can be regarded as an advantage from the toxicity viewpoint. No vapor loss may indicate a less hazardous work environment.

EXAMPLE 4-7

The following experiments were carried out in order to help understand the chemical nature of DMAEMA and MAA reaction product. The materials were added in the order listed at room temperature.

| | Example 4 | Example 5 | Example 6 | Example 7 |
|---|---|---|---|---|
| Materials | | | | |
| | (1) 2cc Distilled Water | 2cc Distilled Water | 2cc Distilled Water | The solution from Ex. 6 |
| | (2) 1cc MAA | 1cc DMAEMA | 1cc DMAEMA-MAA 1:1 mole reaction product | Add 10 drops of 50% NaOH solution. |
| Results | | | | |
| Solubility: | Clear solution | DMAEMA formed separate layer at bottom. | Clear solution | DMAEMA formed separate layer on top. |
| Odor: | Acidic odor | Strong offensive amine odor. | Not detectable | Strong offensive amine odor. |

Example 7 is believed to be a typical acid-base replacement reaction. The strong base NaOH displaced the weak base DMAEMA and formed a new salt ($MAA^-$ $Na^+$). Free DMAEMA was released and thus produced the strong amine odor. Because of the higher density of the new salt, DMAEMA floated to the top instead of being at the bottom as in Example 5. We believe these results and those of the previous examples prove that an amine salt has formed by the reaction of the amine and acid.

EXAMPLES 8-11

The following examples are believed especially illustrative of the relative cure rate of DMAEMA-MAA amine salt compared with DMAEMA and MAA used alone. 100 gram solutions of Examples 8, 9, 10 and 11 were prepared in four ounce amber bottles at room temperature. About one ml of each solution was transferred to 6 x 50 mm clear borosilicate glass culture tubes and exposed to 140 mw/$cm^2$ of visible light supplied by a 150 watt quartz-halogen lamp with a 400-500 nm bandpass filter.

| | Example | | | |
|---|---|---|---|---|
| | 8 (Weight %) | 9 (Weight %) | 10 (Weight %) | 11 (Weight %) |
| 1,6- Hexanediol dimethacrylate (HDDMA) | 99.70 | 98.80 | 98.30 | 91.70 |
| Camphoroquinone (CQ) | 0.30 | 0.30 | 0.30 | 0.30 |
| DMAEMA | - | 0.90 | - | - |
| MAA | - | - | - | 0.50 |
| DMAEMA-MAA (prepared according to Example 2) | - | - | 1.40** | - |
| Visible Light Cure Results | | | | |
| 0 seconds | liquid | liquid | liquid | liquid |
| 50 seconds | liquid | soft gel | rigid semi-solid | liquid |
| 60 seconds | liquid | soft gel | rigid solid | liquid |
| 120 seconds | liquid | rigid solid | rigid solid | liquid |

** This 1.40 weight percent DMAEMA-MAA was derived from 0.50 MAA and 0.90 DMAEMA

Example 8 containing camphoroquinone initiator did not cure after 2 minutes exposure to light in the absence of a photoaccelerator. Example 9 with DMAEMA as photoaccelerator cured after 120 seconds but not after 60 seconds. Example 10 with an equimolar amount of DMAEMA-MAA cured to a rigid solid in 60 seconds. MAA did not act as a photoaccelerator as shown by the lack of cure of Example 11. It was concluded from this experiment that the cure rate of camphoroquinone initiated photopolymerizations were accelerated by DMAEMA-MAA up to twice as fast as with DMAEMA.

DMAEMA-MAA is a difunctional methacrylate as well as a photoaccelerator capable of polymerization with the addition of initiator. The following example is illustrative of the homopolymerization of DMAEMA-MAA.

## EXAMPLE 12

0.3 g camphoroquinone was dissolved in 99.7 grams of DMAEMA-MAA prepared as in Example 2 in a 4 oz. amber glass bottle. About one ml of the solution was transferred to a glass culture tube and cured as described in Example 8-11. After 6 minutes of light exposure the solution cured to a clear rigid solid polymer which was soluble in water. When copolymerized with a minor amount of a crosslinking polymer such as 1,6-hexanediol dimethacrylate, the copolymer is water insoluble.

EXAMPLES 13-16

Photoacceleration speed of amines and amine salt was tested as follows:

Solutions containing 100 g 1,6- Hexanediol dimethacrylate, 0.30 g camphoroquinone and 0.68 g amine or amine salt (prepared according to Example 1) were prepared and tested for cure rate.

The gel time was recorded (in seconds) as the time required for the liquid monomer to gel and start to shrink away from the wall of the glass culture tube. The solution was cured as described above.

The results in increasing effectiveness (decreasing gel time) are as follows:

| Example and Amine/Salt | Relative Mole Ratio | Gel Time | Relative Gel Time |
|---|---|---|---|
| Ex. 13 N-Methyldiethanol amine (DMEA) | 1.00 | 135 sec. | 2.37 |
| Ex. 14 Dimethylaminoethyl methacrylate (DMAEMA) | 0.76 | 71 sec. | 1.25 |
| Ex. 15 Dimethylaminoethyl methacrylate-Methacrylic acid salt (DMAEMA-MAA) | 0.48 | 60 sec. | 1.05 |
| Ex. 16 Dimethylamino-neopentyl acrylate-Methacrylic acid salt (DMANPA-MAA) | 0.44 | 57 sec. | 1.00 |

As can be seen from the above data, DMANPA-MAA salt is more than twice as fast as MDEA of the same weight ratio (or only 0.44 mole ratio relative to MDEA). The DMANPA-MAA salt is also about 25% faster than DMAEMA.

EXAMPLES 17-25

The following examples are especially illustrative of the effect of amine and amine salts on the depth of cure of visible light curable compositions.

A pigmented polymer powder blend was prepared from the pigments, fiber, fumed silica and polymer in a V-Cone Blender. Solutions of HDDMA containing CQ and the amine or amine salts (prepared as described in Example 1) were prepared in four ounce amber bottles. 10g of each solution was combined with 10g (grams) of powder blend in a four ounce amber bottle and allowed to gel for two hours before testing. The depth of cure was measured in Teflon molds in a cylindrical cavity 5 mm deep and 8 mm in diameter. The bottom of the cavity was covered with a cellophane sheet backed up by a glass slide. The compositions of Example 17-25 were packed into the mold and covered with a cellophane sheet. The compositions were cured for three minutes by 132 $mw/cm^2$ of visible light supplied by a 150 watt quartz halogen lamp with a 400-500 nm band-pass filter. The sample was pushed out of the mold and the uncured material was scraped off the bottom with a knife. The bottom was smoothed with 600 grit sandpaper. The height of the cylinder was measured by a micrometer to 0.01 mm as the depth of cure.

DEPTH OF CURE OF COMPOSITIONS ACCELERATED WITH

AMINES OR AMINE SALTS

| | Example 17 | Example 18 | Example 19 | Example 20 |
|---|---|---|---|---|
| Poly (methyl methacrylate-co-ethylene dimethacrylate 99.08:0.2) | 98.59 pbw | 98.59 pbw | 98.59 pbw | 98.59 pbw |
| Fumed silica Aerosil R972 product of Degussa | 1.09 | 1.09 | 1.09 | 1.09 |
| Red Acetate fiber | 0.16 | 0.16 | 0.16 | 0.16 |
| Pigment (Cromophthal Red BR Ciba Geigy) | 0.002 | 0.002 | 0.002 | 0.002 |
| Pigment (Cromophthal Brown 5R Ciba Geigy) | 0.002 | 0.002 | 0.002 | 0.002 |
| Iron Oxide Yellow | 0.002 | 0.002 | 0.002 | 0.002 |
| HDDMA | 100.00 | 100.00 | 100.00 | 100.00 |
| CQ | 0.30 | 0.30 | 0.30 | 0.30 |
| Amine or amine salt } Amount / Type | 0.90 pbw MDEA | 0.90 pbw DMAEMA | 0.90 pbw DMAEMA-MAA | 0.90 pbw DMANPA |
| Depth of Cure after 3 min exposure | 3.0 mm | 3.5 mm | 3.5 mm | 3.6 mm |

Examples 17-21 show that at the same weight ratio the preferred amine salt DMANPA-MAA gave a depth of cure that was 30% greater than MDEA, and 11.4% greater than DMAEMA and DMAEMA-MAA salt. Examples 22 -25 show additional gains in the depth of cure when the amine salts are used at equimolar ratios.

M07-03-84

## DEPTH OF CURE OF COMPOSITIONS ACCELERATED WITH AMINES OR AMINE SALTS (con't)

| | | Example 21 | Example 22 | Example 23 | Example 24 | Example 25 |
|---|---|---|---|---|---|---|
| Poly (methyl methacrylate-co-ethylene dimethacrylate 99.08:0.2) | | 98.59 pbw | 98.59 pbw | 98.59 pbw | 98.59 pbw | 98.59 pbw |
| Fumed silica Aerosil R972 product of Degussa | | 1.09 | 1.09 | 1.09 | 1.09 | 1.09 |
| Red Acetate fiber | | 0.16 | 0.16 | 0.16 | 0.16 | 0.16 |
| Pigment (Cromophthal Red BR Ciba Geigy) | | 0.002 | 0.002 | 0.002 | 0.002 | 0.002 |
| Pigment (Cromophthal Brown 5R Ciba Geigy) | | 0.002 | 0.002 | 0.002 | 0.002 | 0.002 |
| Iron Oxide Yellow | | 0.002 | 0.002 | 0.002 | 0.002 | 0.002 |
| HDDMA | | 100.00 | 100.00 | 100.00 | 100.00 | 100.00 |
| CQ | | 0.30 | 0.30 | 0.30 | 0.30 | 0.30 |
| Amine or amine salt | Amount | 0.90 pbw | 1.19 pbw | 1.84 pbw | 1.40 pbw | 2.05 pbw |
| | Type | DMANPA-MAA | DMAEMA | DMAEMA-MAA | DMANPA | DMANPA-MAA |
| Depth of Cure after 3 min exposure | | 3.9 mm | 3.5 mm | 3.9 mm | 3.6 mm | 4.0 mm |

EXAMPLES 17-21 show that at the same weight ratio the preferred amine salt DMANPA-MAA gave a depth of cure that was 30% greater than MDEA, and 11.4% greater than DMAEMA and DMAEMA-MAA salt. Examples 22-25 show additional gains in the depth of cure when the amine salts are used at equimolar ratios.

EXAMPLE 26

The color stability of amines and amine salts is illustrated as follows:

Solutions containing 50 g HDDMA, 50 g urethane dimethacrylate (Plex 6661-0 Rohm G.M.B.H), 0.3 g CQ and 5.73 x $10^{-3}$ moles of the amine or amine salt as listed below were prepared as previously described in Examples 13-16. The solutions were cast into Plexiglas molds with cylindrical cavities 3 mm deep and 20 mm in diameter. They were then cured using the light and method described in Examples 17-25 except the cure time was two minutes on each side.

Three cured discs of each sample were then masked half way with aluminum foil and placed in a QUV Weatherometer, manufactured by Q-Panel. The fourth disc was removed and placed in dark storage for comparison. Each weathering cycle in the QUV unit includes 4 hours UV at 60°C, followed by 4 hours condensation at 50°C. The total exposure time is 233-1/2 hours.

The samples were then removed and ranked by the degree of discoloration as observed visually by the unaided human eye. For all samples masked with aluminum foil, no discoloration was observed. The exposed sections showed various degrees of discoloration and were ranked in the following sequence.

| Amine or Amine Salt | Wt. % of Amine or Amine Salt | Degree of Discoloration |
|---|---|---|
| MDEA | 0.68 | Severe |
| DMAEMA | 0.90 | Moderate |
| DMAEMA-MAA Salt | 1.40 | Moderate |
| DMANPA | 1.06 | Light |
| DMANPA-MAA Salt | 1.56 | Light |
| DMANPA-MAA Salt | 0.68 | Light |
| Control - no amine or amine salt | - | Light-V. Light |

As can be seen from the above results, DMANPA-MAA Salt has excellent color stability, while both DMAEMA and MDEA have very poor color stability, with MDEA being the most color unstable.

EXAMPLES 27-70

Examples 27 to 70 especially illustrate the influence on acceleration of the cure rate, as measured by the gel time, of the amine salts compared to the amines for a broad variety of different amines and acids. The examples show that the amine salts gave shorter gel times than the amines at Equimolar concentrations, with the exception of the amine salts of very strong acids. Examples 58 to 70 illustrate amine salts that were not useful as accelerators since they were insoluble in the HDDMA or the amines and acids were incompatable in the test monomer solution.

The amine salts were prepared according to Example 2. The solutions were prepared substantially as in Example 13 except $5.76 \times 10^{-6}$ moles of amine or amine salt were used with the 100 grams of 1,6-hexandiol dimethacrylate and 0.3 g camphoroquinone (CQ).

EXAMPLE 27-31

Gel Times of Amine Salts of Monomeric Amines with Glacial Methacrylic Acid

| Example | Chemical Name of Amine | Chemical Structure of Amine | Mol. Wt. of Amine | Physical form of Amine Salt | Exotherm of Salt Formation | Gel Time (sec) Amine | Gel Time (sec) Amine Salt | Ratio of Gel Time Amine | Ratio of Gel Time Amine Salt |
|---|---|---|---|---|---|---|---|---|---|
| 27 | Dimethylaminoneopentyl acrylate | 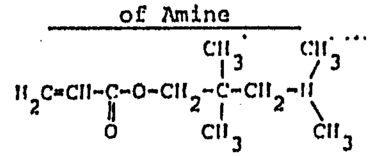 | 185 | liquid | mild | 79 | 48 | 1.65 | 1.00 |
| 28 | 2-N-Morpholinoethyl acrylate | 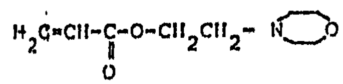 | 185 | liquid | mild | 87 | 63 | 1.38 | 1.00 |
| 29 | 2-N-Morpholinoethyl methacrylate | 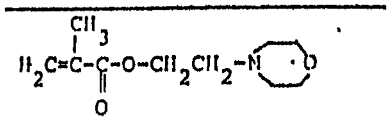 | 199 | liquid | mild | 78 | 56 | 1.39 | 1.00 |
| 30 | Dimethylaminopropyl methacrylamide | 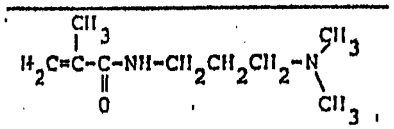 | 170 | liquid | mild | 94 | 64 | 1.47 | 1.00 |
| 31 | Dimethylaminoethyl methacrylate | 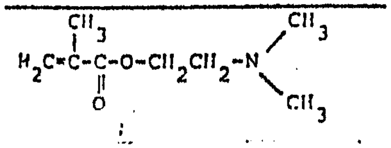 | 157 | liquid | high, fumed | 63 | 51 | 1.24 | 1.00 |

The amine salts have cure rates that are 24% to 65% faster than the amines themselves.

## Examples 32 - 35

### Gel Times of Amine Salts of Dimethylaminoneopentyl Acrylate with Organic Acids

| Example | Chemical Name of Acid | Chemical Structure of Acid | Mol. Wt. of Acid | Physical Form of Amine Salt |
|---|---|---|---|---|
| 32 | Glacial Methacrylic Acid | $CH_3$<br>\|<br>$CH_2=C-COOH$ | 86 | Liquid |
| 33 | Glacial Acrylic Acid | $CH_2=CH-COOH$ | 72 | Initially Liquid then gelled (Insoluble in HDDMA) |
| 34 | β-Carboxyethyl Acrylate | $CH_2=CH-C-O-CH_2CH_2COOH$<br>‖<br>O | 144 | Viscous paste then gelled (Insoluble in HDDMA) |
| 35 | Acetic Acid | $CH_3COOH$ | 60 | Liquid |

The amine salts have cure rates that are 58% to 76% faster than the amines themselves.

Examples 32 - 35 (con't)

Gel Times of Amine Salts of Dimethylaminoneopentyl Acrylate with Organic Acids

| Example | Chemical Name of Acid | Exotherm of Salt Formation | Gel time (sec.) Amine | Gel time (sec.) Amine Salt | Ratio of Gel Time Amine | Ratio of Gel Time Amine Salt |
|---|---|---|---|---|---|---|
| 32 | Glacial Methacrylic Acid | High, fumed | 79 | 48 | 1.65 | 1.00 |
| 33 | Glacial Acrylic Acid | High, fumed | 79 | 45 | 1.76 | 1.00 |
| 34 | β-Carboxyethyl Acrylate | Mild, fumed | 79 | 50 | 1.58 | 1.00 |
| 35 | Acetic Acid | High, fumed | 79 | 50 | 1.58 | 1.00 |

The amine salts have cure rates that are 58% to 76% faster than the amines themselves

EXAMPLES 36-44

Gel Times of Amine Salts of Glacial Methacrylic Acid with Saturated Amines

| Example | Tertiary Amines Used: Chemical Name | $R_1$ * | $R_2$ * | $R_3$ * | Gel Time (Seconds): Amine | Gel Time (Seconds): 1:1 Amine-MAA Salt | Ratio of Gel Time: Amine | Ratio of Gel Time: Amine-MAA Salt |
|---|---|---|---|---|---|---|---|---|
| 36 | N-Ethyldiethanolamine | $-C_2H_5$ | $-CH_2CH_2OH$ | $-CH_2CH_2OH$ | 150 | 55 | 2.7 | 1.0 |
| 37 | N-Methyldiethanolamine | $-CH_3$ | $-CH_2CH_2OH$ | $-CH_2CH_2OH$ | 135 | Self Gelled, not tested | - | - |
| 38 | N,n-butyldiethanolamine | $-(CH_2)_3CH_3$ | $-CH_2CH_2OH$ | $-CH_2CH_2OH$ | 120 | 60 | 2.0 | 1.0 |
| 39 | Tributylamine | $-(CH_2)_3CH_3$ | $-(CH_2)_3CH_3$ | $-(CH_2)_3CH$ | 150 | 60 | 2.5 | 1.0 |
| 40 | N,N-Dimethylethanolamine | $-CH_3$ | $-CH_3$ | $-CH_2CH_2OH$ | 75 | 62 | 1.2 | 1.0 |
| 41 | 1-Dimethylamino-2-propanol | $-CH_3$ | $-CH_3$ | $-CH_2CH-OH$<br>$CH_3$ | 80 | 55 | 1.4 | 1.0 |
| 42 | 1-Diethylamino-2-propanol | $-C_2H_5$ | $-C_2H_5$ | $-CH_2CH-OH$<br>$CH_3$ | 170 | 70 | 2.4 | 1.0 |
| 43 | 2-Dimethylamino-2-methyl-1-propanol | $-CH_3$ | $-CH_3$ | $CH_3$<br>$-C-CH_2OH$<br>$CH_3$ | 135 | 80 | 1.7 | 1.0 |
| 44 | None | - | - | - | 6 min. 22 sec. (382 sec.) | - | - | - |

The amine salts have cure rates that are 20% to 170% faster than the amines themselves.

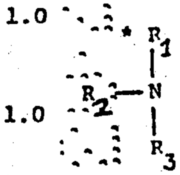

## Examples 45 - 57

### Gel Times of Amine Salts of Dimethylaminoethyl Methacrylate with Various Acids

| mple | Chemical Name of Acid | Chemical Structure of Acid | Mol. Wt. of Acid | Phisical Form of Amine Salt |
|---|---|---|---|---|
| 5 | β-Carboxyethyl acrylate | $CH_2=CH-\underset{O}{\overset{}{C}}-O-CH_2CH_2-COOH$ | 144 | Viscous liquid then gelled |
| 6 | Glacial acrylic acid | $CH_2=CH-COOH$ | 72 | Clear liquid then gelled |
| 7 | Glacial methacrylic acid | $CH_2 = \overset{CH_3}{C} - COOH$ | 86 | Clear liquid |
| 8 | Adipic acid * | $HOOC(CH_2)_4COOH$ | 146 | Viscous liquid |
| 9 | n-butyric acid | $CH_3CH_2CH_2COOH$ | 88 | Clear liquid |
| 0 | Hexanoic acid | $CH_3(CH_2)_4COOH$ | 116 | Clear liquid |
| 1 | Lauric acid | $CH_3(CH_2)_{10}COOH$ | 200 | Clear liquid |
| 2 | Acetic acid | $CH_3COOH$ | 60 | Clear liquid |
| 3 | Adipic acid ** | $HOOC(CH_2)_4COOH$ | 146 | Viscous liquid |
| 4 | Control, no acid, pure DMAEMA | - | - | - |
| 5 | Perfluorobutyric acid (3M FC-23) | $CF_3CF_2CF_2COOH$ | 214 | Clear viscous liquid |

Examples 45 - 57 (con't)

Gel Times of

Amine Salts of Dimethylaminoethyl Methacrylate with Various Acids

| Example | Chemical Name of Acid | Exotherm of Salt Formation | Gel Time (Seconds) | Ratio of Gel Time | |
|---|---|---|---|---|---|
| | | | | DMAEMA only | DMAEMA Acid Salt |
| 45 | β-Carboxyethyl acrylate | mild, fumed | 47 | 1.34 | 1.00 |
| 46 | Glacial acrylic acid | high, fumed | 48 | 1.31 | 1.00 |
| 47 | Glacial methacrylic acid | high, fumed | 51 | 1.24 | 1.00 |
| 48 | Adipic acid * | very slight | 51 | 1.24 | 1.00 |
| 49 | n-butyric acid | mild | 52 | 1.21 | 1.00 |
| 50 | Hexanoic acid | mild | 52 | 1.21 | 1.00 |
| 51 | Lauric acid | very slight | 52 | 1.21 | 1.00 |
| 52 | Acetic acid | high, fumed | 53-1/2 | 1.18 | 1.00 |
| 53 | Adipic acid ** | very slight | 53-1/2 | 1.18 | 1.00 |
| 54 | Control, no acid, pure DMAEMA | - | 63 | 1.00 | - |
| 55 | Perfluorobutyric acid (3M FC-23) | very high-a lot of fumes | 128 | 1.00 | 2.03 |

EXAMPLES 45-57 (Continued)

Gel Times of

Amine Salts of Dimethylaminoethyl Methacrylate with Variuos Acids

| Example | Chemical Name of Acid | Chemical Structure of Acid | Mol. Wt. of Acid | Physical Form of Amine Salt |
|---|---|---|---|---|
| 56 | Perchloric acid | $HClO_4$ | 100 | liquid |
| 57 | Trifluoromethane sulfonic acid (3M FC-24) | $CF_3SO_3H$ | 150 | Brown viscous liquid |

With the exception of the very strong acids of Examples 55-57 the amine salts have cure rates that are 18% to 34% faster than DMAEMA alone.

M07.03.84

EXAMPLES 45-57 (Continued)

Gel Timse of

Amine Salts of Dimethylaminoethyl Methacrylate with Variuos Acids

| Dxample | Chemical Name of Acid | Exotherm of Salt Formation | Gel Time (Seconds) | Ratio of Gel Time | |
|---|---|---|---|---|---|
| | | | | DMAEMA only | DMAEMA Acid Salt |
| 56 | Perchloric acid | very high-a lot of fumes | semi-solid at 300 | 1.00 | > 4.76 |
| 57 | Trifluoromethane sulfonic acid (3M FC-24) | Very high-a lot of fumes | soft gel at 480 | 1.00 | > 7.62 |

* 1 mole Adipic Acid reacted with 1 mole DMAEMA

** 1 mole Adipic Acid reacted with 2 mole DMAEMA

With the exception of the very strong acids of Examples 55-57 the amine salts have cure rates that are 18% to 34& faster than DMAEMA alone.

Examples 58 - 70

Acids Which do not Form Useable Salts with DMAEMA in HDDMA

| Example | Chemical Name of Acid | Chemical Structure of Acid | Mol.Wt. of Acid | Physical Form of Amine Salt |
|---|---|---|---|---|
| 58 | Oxalic Acid | HOOC--COOH | 90 | Pasty solid |
| 59 | Oxalic Acid, dihydrate | $HOOC-COOH\cdot 2H_2O$ | 126 | Pasty solid |
| 60 | Trimellitic Anhydride | 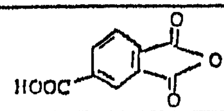 | 192 | Moist pellets |
| 61 | Picric Acid | 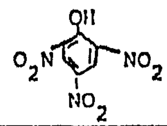 | 229 | Rigid lumps, excess liquid |
| 62 | Citrazinic Acid | 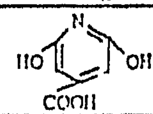 | 155 | Dark rigid solid, excess liquid |
| 63 | 2-Thiobarbituric Acid | 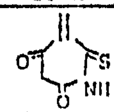 | 144 | Solid - liquid separate phase |
| 64 | Methyl Red | 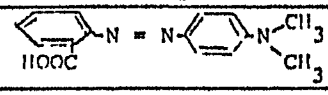 | 269 | Deep violet paste |
| 65 | Ethylene diamine tetraacetic acid(EDTA) | 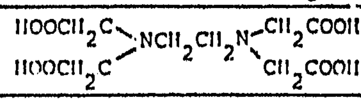 | 292 | Solid - liquid phase separation |
| 66 | Molybdic Acid | $MoO_3$ | 144 | Solid - liquid phase separation |
| 67 | Barbituric Acid | 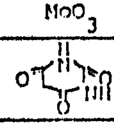 | 128 | Paste |
| 68 | Sulphuric Acid | $H_2SO_4$ | 98 | Gelled semi-solid |
| 69 | Sulfanilic Acid | 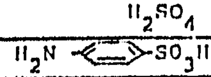 | 173 | Dark gelled Solid |
| 70 | Ascorbic Acid | 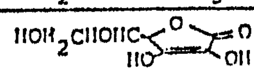 | 176 | Solid - liquid phase separation |

The acids in these examples either did not react or formed reaction products insoluble in HDDMA

Examples 58 - 70

Acids Which do not Form Useable Salts with DMAEMA in HDDMA

| Example | Chemical Name of Acid | Exotherm of Salt Formation | Comments |
|---|---|---|---|
| 58 | Oxalic Acid | High | Did not dissolve in HDDMA |
| 59 | Oxalic Acid, dihydrate | High | Did not dissolve in HDDMA |
| 60 | Trimellitic Anhydride | Mild | Did not dissolve in HDDMA |
| 61 | Picric Acid | None | Did not dissolve in HDDMA |
| 62 | Citrazinic Acid | None | Did not dissolve in HDDMA |
| 63 | 2-Thiobarbituric Acid | None | Did not dissolve in HDDMA |
| 64 | Methyl Red | None | Did not dissolve in HDDMA |
| 65 | Ethylene diamine tetraacetic acid(EDTA) | None | Did not dissolve in HDDMA |
| 66 | Molybdic Acid | None | Did not dissolve in HDDMA |
| 67 | Barbituric Acid | Mild | Did not dissolve in HDDMA |
| 68 | Sulphuric Acid | Very high - a lot of fumes | Did not gel after 3 min. light exposure |
| 69 | Sulfanilic Acid | Mild | Did not dissolve in HDDMA |
| 70 | Ascorbic Acid | None | Did not dissolve in HDDMA |

The Acide in these examples either did not react or formed reaction products insoluble in HDDMA

## EXAMPLES 71-75

Examples 71-75 illustrate that the invention is generally applicable to various monomers and oligomers that can be subject to photocuring. Each Example was carried out by initially charging the liquid components into an amber glass bottle and then adding the camphoroquinone (CQ) into the bottle and shaking the bottle on a mechanical shaker until the CQ had dissolved. Then the bottle was removed from the shaker and the viscous monomers or oligomers were added and the bottle was heated for one half hour at 60°C and then stirred until uniform. The curing was carried out as in Examples 17-25.

### Example 71

| | |
|---|---|
| 79.65% | Urethane dimethacrylate (Plex 6661-0 Rohm GmbH) |
| 18.77 | HDDMA |
| 0.26 | CQ |
| 1.32 | DMANPA-MAA salt prepared according to Example 1 |

Light cured in 10 seconds, useful as coating.

### Example 72

| | |
|---|---|
| 85.71% | a non-volatile diacrylate ester of a bisphenol-A type epoxy resin (Celrad 3500 Celanese Plastics and Specialty Co.) |
| 9.52 | Sipomer β-CEA β-Carboxyethyl acrylate from Alcolac, Inc |
| 3.14 | dicyclopentenyloxyethyl methacrylate (QM657 Rohm & Haas Co.) |
| 0.28 | CQ |
| 1.33 | DMAEMA-MAA salt prepared according to Example 2 |

Useful as coating. Light cured in less than 3 minutes.

### Example 73

| | |
|---|---|
| 60.08% | a medium viscosity second generation aliphatic diacrylate (Photomer 4083 Diamond Shamrock Corp.) |
| 35.06 | Plex 6661-0 |
| 0.30 | CQ |
| 1.56 | DMANPA-MAA salt prepared according to Example 1 |
| 3.00 | Stearyl methacrylate |

Useful as coating. Light cured in less than 2 minutes.

EXAMPLES 71-75 (Continued)

Example 74

58.94% an acrylated urethane oligomer (Uvithane 893 Morton-Thiokol)
39.29 Diethylene Glycol dimethacrylate
0.29 CQ
1.48 DMANPA-MAA salt prepared according to Example 1

Useful as coating. Light cured in 1 minute.

Example 75

92.71% Trimethylolpropane trimethacrylate
5.56 Pentaerythritol tetra-3-mercaptopropionate
0.28 CQ
1.45 DMANPA-MAA salt prepared according to Example 1

Useful as coating. Light cured in 1 minute.

EXAMPLE 76

A composition that is moldable to a denture base form and hardenable with visible light was prepared from the following ingredients:

| Percent by Weight of Total Composition | |
|---|---|
| 39.44 | Urethane dimethacrylate (Reaction product of hydroxyethyl methacrylate and 2,2,4-trimethylhexyl-1,6-diisocyanate) |
| 2.57 | HDDMA |
| 0.13 | CQ |
| 0.59 | DMAEMA-MAA salt prepared as in Example 2 |
| 42.10 | poly (methyl methacrylate-co-ethylene dimethacrylate 99.8:0.2) a polymer supplied by L. D. Caulk Co (Polymer is in substantially spherical beads the shape resulting from its suspension polymerization. At least 50% by weight of the beads have average diameters of less than 50 microns. The polymer was prepared from methyl methacrylate and ethylene dimethacrylate by suspension polymerization*). |
| 0.07 | red acetate fibers |
| 0.03 | pigments |
| 15.07 | fumed silica inorganic filler (Aerosil R972 a product of Degussa) |

First CQ was dissolved in HDDMA and then mixed with the amine salt and urethane dimethacrylate. The resulting liquid solution was charged to a double planetary mixer heated to 45°C and mixed under 20 mm Hg pressure. Next the polymer

*The polymer was prepared according to the teaching of U.S. Serial No. 318,356, filed Nov. 5, 1981, which was a Continuation of U.S. Serial No. 008,507, filed Feb. 1, 1977.

with the pigments and fibers previously blended in a V-Cone Blender was added and mixed under 20 mm pressure. The temperature was increased to 55°C and the fumed silica was added in three increments of about equal size and mixed under 130 mm pressure each time. This produced the visible light curable (VLC) putty-paste of this invention.

The VLC putty was molded into a sheet 3.5" x 2.5" x 0.10" in a hydraulic press. The sheet was adapted as a baseplate to a stone model (coated with separator) made from an impression of the mouth. The baseplate was trimmed and then cured in two minutes on a turntable rotating under four 150 watt quartz-halogen lamps with a 400-500 nm band-pass filter. The light flux varied from 100-130 $mw/cm^2$ on the surface of the baseplate. Additional VLC putty was rolled into rope 0.25" in diameter. The rope was adapted around the ridge of the baseplate and a full arch of acrylic plastic teeth coated with a bonding agent was press-positioned in the rope. The teeth were further positioned in an articulator and then fixed in position by a two-minute light cure. Next the facial and lingual aspects of the denture were finished with additional rope. The denture was coated with an oxygen barrier coating then light-cured for two minutes. The denture was removed from the model, coated with an oxygen barrier coating and then cured two minutes. The denture was polished by the usual means, cleaned in an ultrasonic bath and rinsed. The denture was inserted into a human mouth, found in the reported opinion of the clinician, to be equal to or superior to dentures made with conventional denture materials by conventional methods.

EXAMPLE 77

100 g of a visible light curable composition useful as opaquing medium for dental alloys was prepared by dissolving and mixing the following ingredients:

| | |
|---|---|
| 50.91% | acrylate urethane oligomer (Urethane 782) |
| 30.46 | dicyclopentenyloxyethyl methacrylate (QM-657 Rohm & Haas Co.) |
| 9.73 | MAA |
| 3.05 | $TiO_2$ |
| 3.05 | fumed silica (Aerosil R972) |
| 0.36 | CQ |
| 2.44 | DMAEMA-MAA salt, prepared as in Example 2 |

The resulting composition was a viscous, yet flowable opaque white liquid.

Rectangular tabs measuring about 0.35" x 1.00" x 0.05" were cast from a nickel-chromium based dental alloy (sold under the trademark BIOBOND, manufactured by Dentsply International Inc.). They were ground and sandblasted with aluminum oxide and then tin plated for 30 seconds with Tin Alkaline 5001, a tin plating solution supplied by Liquid Development Corp., and oxidized with 5% hydrogen peroxide solution for 15 seconds. After drying with Dust Chaser (dichlorodifluoromethane), supplied by VWR Scientific Inc., the above opaquing medium was brushed onto half of the metal tab and then cured for 3 minutes under the light as in Examples 17-25. A visible light curable dental composite (sold under the trademark PRISMA-FIL, manufactured by The L. D. Caulk Co., a division of Dentsply International Inc.), was then applied over the cured opaquing medium, and cured for an additional 3 minutes. The above cured laminates were conditioned in 37°C deionized water for 40 hours. After that, the samples were subjected to boiling water for 30 seconds, followed by ice water for 30 seconds thermocycling for 30 cycles. An aluminum tab, measuring about 0.45" x

1.25" x 0.10" was glued over the previously referred to dental composite surface with a structural adhesive (Versilok 204, manufactured by Lord Corp.). The samples were then subjected to lap shear tensile bond strength test at 0.05 inch per minute head speed, using an Instron Universal Testing Machine. The measured failure strength was 618 psi. The failure occurred at the interface between aluminum and the structural adhesive, indicating that the bond strength was more than the 618 psi.

Examples 8-11 and 13-26 are believed to have clearly demonstrated that the DMANPA-MAA is a much more efficient and color stable photoaccelerator than either MDEA or DMAEMA.

The preferred organic tertiary amine salt of the present invention is a photoaccelerator that offers real advantages in visible light cured dental and industrial products that require faster cure and/or greater depth of cure. The preferred amine salt is a difunctional monomer and coreactive with the system polymerized. Because it is coreactive at both its amine moiety and acid moiety it is bound in the polymer system and thus not generally subject to being leached out under the harsh fluctuations between acid and alkaline materials that are constantly placed in the oral environment during the consumption of foods.

It was surprising that the formation of the salt did not block the action of the tertiary amine group in the tertiary amine salts when used as photoaccelerators. It was even more surprising that the salt acts as a superior accelerator to the tertiary amines themselves.

The foregoing description illustrates preferred embodiments of the invention. However, concepts employed may, based upon such description, be employed in other embodiments without departing from the scope of the invention. Accordingly, the following claims are intended to protect the invention broadly, as well as in the specific forms shown herein.

IT IS CLAIMED:

1. A method of modifying the polymerization of a polymerizable composition comprising incorporating in said composition a neopentyl acrylate amine moiety and an acid moiety.

2. The method of modifying the polymerization of a polymerizable composition of Claim 1 wherein said neopentyl acrylate amine moiety and said acid moiety are associated in a salt.

3. The method of modifying the polymerization of a polymerizable composition of Claim 2 wherein said salt has the formula.

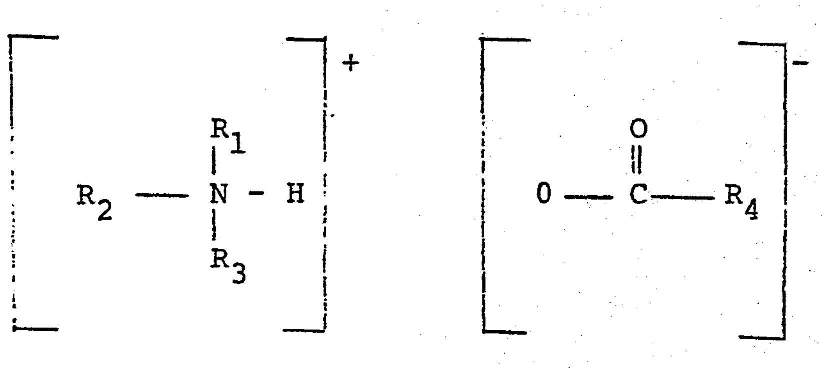

wherein $R_1$ and $R_2$ are alkyl or substituted alkyl radicals having from 1-4 C atoms and $R_3$ is neopentyl acrylate having a total of 8 to 10 C and $R_4$ is a vinyl having from 3 to 6 C.

4. The method of modifying the polymerization of a polymerizable composition of Claim 3 wherein $R_1$ and $R_2$ are methyl, $R_3$ is neopentyl acrylate having 8 C and $R_4$ is $-\overset{CH_3}{\overset{|}{C}}=CH_2$, and wherein said polymerizable composition comprising at least one additional acrylate that polymerizes by addition polymerization, a diketone initiator and is visible light curable.

5. A polymerizable composition comprising

(1) organic amine salt compound and

(2) polymerization initiator compound

6. The polymerizable composition of Claim 5 comprising at least one polymerizable organic compound other than said organic amine salt compound, said organic amine salt compound being chosen from the group consisting of polymerizable, and unpolymerizable organic amine salts and mixtures thereof.

7. The polymerizable composition of Claim 6 wherein said polymerization initiator compound is a radiation activatable compound.

8. The polymerizable composition of Claim 7 wherein additional said polymerization initiator compound is present and it is heat activatable compound.

9. The polymerizable composition of Claim 7 wherein said polymerizable composition is storage stable at 20$^{\circ}$C for at least one week under light free conditions and said radiation activatable compound comprising an actinic light activatable compound.

10. The polymerizable composition of Claim 6 wherein said organic amine salt compound comprising the salt of an organic acid.

11. The polymerizable composition of Claim 7 wherein said radiation activatable compound comprising an actinic light activatable compound and said organic amine salt compound comprising the formula

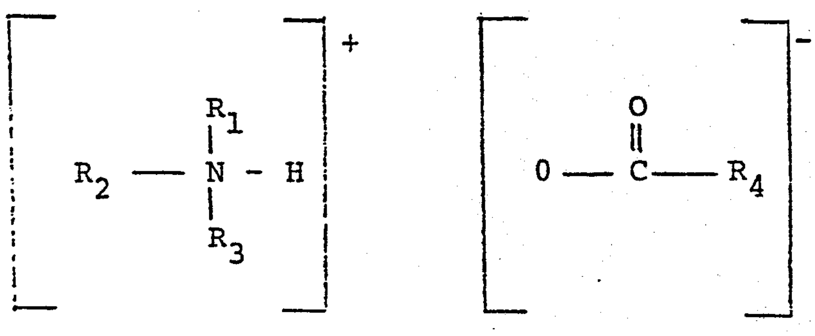

wherein $R_1$, $R_2$ and $R_3$ are each either an organic radical or H and $R_4$ is an organic radical or H.

12. The polymerizable composition of Claim 11 wherein the polymerizable system consists of said organic amine salt compound, said radiation activatable compound and said at least one polymerizable organic compound and said organic amine salt compound is present in an amount of about 0.05 to about 99 percent by weight of said polymerizable system and comprising the stated general formula and wherein at least one of $R_1$, $R_2$ and $R_3$ contains the group $-CH_2-\overset{CH_3}{\underset{CH_3}{C}}=$ ; said at least one polymerizable organic compound is present in an amount of about 1 to about 99.9 percent by weight of said polymerizable system and comprising an ethylenically unsaturated compound; said radiation activated compound is present in an amount of about .001 to about 10 percent by weight of said polymerizable system; and wherein said polymerizable composition comprising filler present

in an amount of about 0 to about 70 percent on a volume basis based on the total volume of said polymerizable composition.

13. The polymerizable composition of Claim 12 as a dental composition for end use in the oral environment wherein said organic amine salt compound is present in an amount of about 0.1 to about 10 percent by weight of said polymerizable system and comprising the stated general formula and wherein $R_1$ and $R_2$ are alkyl or substituted alkyl radicals, $R_3$ is a saturated or unsaturated neopentyl radical and at least one of $R_1$, $R_2$ or $R_3$ is unsaturated and $R_4$ is unsaturated; said at least one polymerizable organic compound is present in an amount of about 85 to about 99.9 percent by weight of said polymerizable system and comprising an acrylate polymerized predominately through addition polymerization; said radiation activated compound comprising diketone present in an amount of about 0.01 to about 5 percent by weight of said polymerizable system and wherein said polymerizable composition has a filler content of about 35 to about 70 percent on a volume basis based on the total volume of said polymerizable composition.

14. The polymerization composition of Claim 13 wherein said organic amine salt compound is present in an amount of about 0.3 to about 5 percent by weight of said polymerizable system and comprising the non aqueous reaction product of 3-(N,N-dimethylamino)-2,2-dimethylpropyl acrylate with methacrylic acid; said at least one polymerizable organic

compound is present in an amount of about 94.5 to about 99.6 percent by weight of said polymerizable system and is chosen from the group consisting of mono and multifunctional acrylates and said diketone is present in an amount of about 0.1 to about 0.5 percent by weight of said polymerizable system and wherein said composition has a filler content of about 45 to about 70 percent on a volume basis based on the total volume of said polymerizable composition.

15. The method of forming an organic amine salt of an acid comprising;

(1) preparing separate feed stocks of organic amine and acid;

(2) bringing said feed stocks together under conditions such that one of said feed stocks is below about $0^{o}C$ at the time of combining.

16. The method of forming an organic amine salt of an acid of Claim 15 wherein both said organic amine feed stock and said acid feed stock are cooled to at least about $0^{o}C$ before they are combined.

17. The method of forming an organic amine salt of an acid of Claim 16 wherein said acid is an organic acid, said cooling is to a degree that solidifies said organic acid and said organic acid and said organic amine are brought together in reaction with said organic acid initially in a solid state and said reaction is exothermic.

18. The method of forming an organic amine salt of an acid of Claim 17 wherein said reactants have the formulas

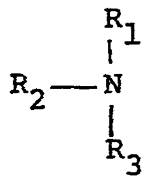

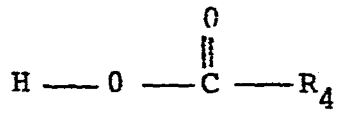

wherein $R_1$, $R_2$ and $R_3$ are each either an organic radical or H and $R_4$ is an organic radical or H.

19. The method of forming an organic amine salt of an acid of Claim 18 wherein $R_3$ is neopentyl acrylate radical having 8 to 10 C and $R_4$ contains a vinyl group.

20. The method of forming an organic amine salt of an acid of Claim 19 wherein $R_1$ and $R_2$ are alkanes having 1 to 2 C atoms and $R_3$ is a neopentyl acrylate radical having 8 to 9 C and $R_4$ is 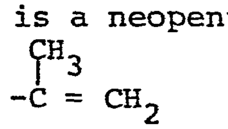 and said organic amine salt is formed in non-aqueous conditions.

21. A composition of matter comprising a salt comprising

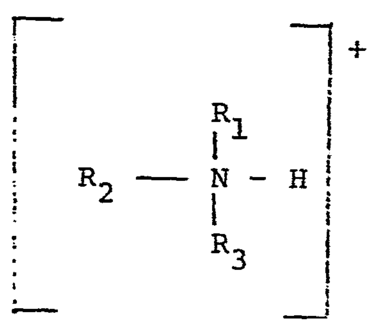

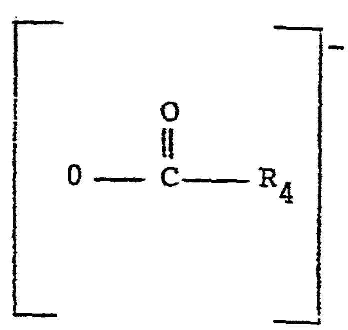

wherein $R_1$, $R_2$ and $R_3$ are each either an organic radical or H and at least one of $R_1$, $R_2$ and $R_3$ contains the group

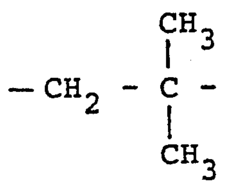

and $R_4$ is an organic radical or H.

22. The composition of matter of Claim 21 wherein said salt on treatment of one ml of salt in 2 ml of water in a glass test tube with ten drops of 50% NaOH aqueous solution results in the separation of an amine layer on top which exhibits a characteristic strong offensive amine odor.

23. The composition of matter of Claim 21 wherein said salt is further defined as: $R_3$ is a neopentyl acrylate radical with 8 to 10 C and $R_4$ contains a vinyl group.

24. The composition of matter of Claim 23 wherein said salt is further defined as: $R_1$ and $R_2$ are alkanes having 1 to 6 C and $R_3$ is a neopentyl acrylate radical having 8-9 C and $R_4$ is

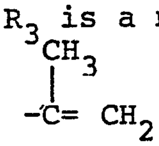

25. A polymerizable composition comprising:

(1) addition polymerizable compound;

(2) photoinitiator; and

(3) photoaccelerator comprising salt of organic amine and organic acid.

26. The polymerizable composition of Claim 25 wherein said addition polymerizable compound comprising acrylic or substituted acrylic monomer and said photoaccelerator salt comprising unsaturated organic amine moiety and unsaturated organic acid moiety.

27. The polymerizable composition of Claim 26 wherein said organic amine moiety comprising tertiary amine.

28. The polymerizable composition of Claim 27 wherein said tertiary amine moiety is a neopentyl acrylate amine and said acid moiety is a methacrylic acid, and said photoinitiator comprising diketone.

European Patent Office

0126866

Application number

# EUROPEAN SEARCH REPORT

EP 84 10 2448

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | US-A-3 950 398 (MAX KLEIN)<br>* Column 8, lines 34-36; claim * | 1-3,15-23 | C 08 F 20/36<br>C 08 F 299/04<br>C 07 C 93/193<br>A 61 K 6/08 |
| | --- | | |
| Y | EP-A-0 068 225 (BASF)<br>* Claims * | 1-3 | |
| | --- | | |
| A | EP-A-0 011 734 (BAYER) | | |
| | --- | | |
| A | EP-A-0 027 850 (RÖHM) | | |
| | ----- | | |

| TECHNICAL FIELDS SEARCHED (Int. Cl. 3) |
|---|
| C 08 F<br>G 03 C<br>A 61 K<br>C 07 C |

The present search report has been drawn up for all claims

| Place of search | Date of completion of the search | Examiner |
|---|---|---|
| THE HAGUE | 08-08-1984 | LENSEN H.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82